# EUROPEAN PATENT SPECIFICATION

(11) **EP 1 360 504 B1**
(45) Date of publication and mention of the grant of the patent: **13.09.2006**
(21) Application number: 02715378.2
(22) Date of filing: 17.01.2002
(51) Int. Cl.: G01N 33/68

(54) **METHOD FOR SCREENING COMPOUNDS FOR ACTIVITY IN TREATING AN OSTEOCLAST RELATED BONE DISEASE**
VERFAHREN ZUM SCREENING VON VERBINDUNGEN AUF AKTIVITÄT BEI DER BEHANDLUNG EINER MIT OSTEOKLASTEN ZUSAMMENHÄNGENDEN KNOCHENERKRANKUNG
TECHNIQUE DE CRIBLAGE DE COMPOSES EN VUE D'IDENTIFIER UNE ACTIVITE CONVENANT POUR LE TRAITEMENT D'UN OSTEOCLASTE LIE A UNE MALADIE OSSEUSE

(30) Priority: 23.01.2001 DK 200100118; 05.02.2001 US 265874 P
(43) Date of publication of application: 12.11.2003
(73) Proprietor: Pharmos Bioscience A/S, 2730 Herlev (DK)
(72) Inventor: HEEGAARD, Anne Marie, DK-2730 Herlev (DK); ENGSIG, Michael Thyrring, DK-2730 Herlev (DK); MADSEN, Lars Siim, DK-2750 Ballerup (DK); JENSEN, Bo Skaaning, DK-2750 Ballerup (DK); CHRISTOPHERSEN, Palle, DK-2750 Ballerup (DK); STAHLHUT, Martin, DK-2730 Herlev (DK); KARSDAL, Morten Asser, DK-2730 Herlev (DK)
(74) Representative: Smart, Peter John
(86) International application number: PCT/DK2002/000037
(87) International publication number: WO 2002/059356

(56) References cited:
- WO-A-00/24707
- WO-A-02/059612
- S.H.S. PEARCE: "Straightening out the renal tubule: advances in the molecular basis of the inherited tubulopathies" QJ MED, vol. 91, 1998, pages 5-12, XP002902727
- UWE KORNAK ET AL: "Loss of the CIC-7 chloride Channel leads to Osteopetrosis in Mice and Man " CELL, vol. 104, 26 January 2001 (2001-01-26), pages 205-215, XP002902614
- ERNA CLEIREN ET AL: "Albers-Schönberg disease (autosomal dominant osteopetrosis, type II) results from mutations in the CICN7 chloride channel gene" MOLECULAR GENETICS, vol. 10, no. 25, 11 October 2001 (2001-10-11), pages 2861-2867, XP002902728
- PAUL H.SCHLESINGER ET AL: "Characterization of the Osteoclast Ruffled Border Chloride Channel and its Role in Bone Resorption" THE JOURNAL OF BIOLOGICAL CHEMISTRY, vol. 272, no. 30, 25 July 2002 (2002-07-25), pages 18636-18643, XP002902729
- SILKE BRANDT ET AL: "C1C-6 and C1C-7 are two novel broadly expressed members of the CLC chloride channel family" FEBS LETTERS, vol. 377, 1995, pages 15-20, XP002902613

## Description

The present invention relates to a method for screening compounds for activity in treating an osteoclast related bone disease.

### BACKGROUND ART

Cells are protected from their external environment by a cell membrane. In order to communicate with the outside world, cells have developed transmembrane proteins, which bind external molecules to activate intracellular signal transduction mechanisms, or transport solutes in or out of the cell. Ion channels are transmembrane proteins, which catalyse the transport of inorganic ions across cell membranes.

The ion channels participate in processes as diverse as generating and timing of action potentials, synaptic transmission, secretion of hormones, contraction of muscles, signal transduction, etc. Ion channels can be divided according to the ions they conduct, i.e. K⁺ channels, Na⁺ channels and Cl⁻ channels. Cl⁻ channels are probably found in every cell, from bacteria to mammals. Their physiological tasks range from cell volume regulation to stabilization of the membrane potential, transepithelial or transcellular transport and acidification of intracellular organelles. These different functions require the presence of many distinct chloride channels, which are differentially expressed and regulated by various stimuli.

### Osteoporosis and Cl⁻ channels

A healthy bone results from a balance between ongoing bone formation and resorption processes. Bone formation is dependent on osteoblasts activity while osteoclasts are involved in bone resorption.

It is well known that the pathogenesis of osteoporosis can result from an imbalance between bone formation and resorption as a result of dominant osteoclasts activity.

Osteoclasts attach bone whereby a bone resorption compartment is generated as a cavity between the osteoclast and the bone. This compartment is tightly sealed and isolated from the pericellular compartment. Upon bone attachment the osteoclast plasma membrane develops into a ruffled membrane which delimits the bone resorption compartment.

The bone resorption process mediated by osteoclasts is highly dependent on acidification of the bone resorption compartment. A H⁺-ATPase transports protons from osteoclast cytosol across the ruffled membrane into the bone resorption compartment. Cl⁻ ions from osteoclast cytosol passively follow the protons through anion channels in the ruffled membrane to the bone resorption compartment. This coordinated transport of protons and C1 ions (generating HCl) acidifies the bone resorption compartment resulting in dissolution of the mineral bone components and consequent exposure of the protein part of the action of osteoclast proteases.

Schlesinger, P.H. et al (J. Biol.Chem (1997) 272, 18636-18643) disclose that an anion channel expressed in the osteoclast is related to the bovine p64 C1 channel, but the molecular structure of the ruffled membrane C 1 channel is not known.

WO 00/24707 discloses compounds useful as chloride channel blockers.

However, there is a strong interest in the provision of more effective and selective compounds with fewer side effects for the treatment of patients with an osteoclast related bone disease, such as osteoporosis.

WO-A-0024707 discloses pharmaceutical compounds for use in treating osteoclast associated disorders and other conditions and discloses measuring the ability of compounds to block one or more chloride channels in erythrocytes and that the compounds can be used in the treatment of osteoclast related bone disease in a subject.

Brandt et al, FEBS lettes, 1995, 377: 15-20, teaches that chloride channels that are members of the ClC family are expressed over a broad range of tissues.

### SUMMARY OF THE INVENTION

According to the invention it has now been found that the chloride channels of the ClC family are involved in the resorption of bone. Therefore, chloride channels of the ClC family, such as ClC-3, ClC-6, and ClC-7 constitute molecular targets for the treatment of an osteoclast related bone disease, such as osteoporosis.

Thus, in its first aspect, the invention relates to a method for screening chemical compounds for activity in the treatment, prevention, or alleviation of an osteoclast related bone disease in a subject, which method comprises screening compounds in a test cell so as to select compounds having the ability to block one or more channels of the ClC family.

Preferred methods comprise screening the compounds so as to select compounds having the ability to block one or more of the chloride channels ClC-3, CIC-6 and ClC-7, e.g. for the ability to block ClC-7.

Preferably such a method comprises the following steps:
- providing a test cell comprising one or more chloride channels of the ClC family;
- subjecting the test cell to the action of the chemical compound; and
- measuring the ability of the compound to block the one ore more chloride channels.

A compound identified as a blocker of a chloride chanel of the ClC family by the method as described above or a pharmaceutically acceptable salt or a prodrug therefore may be used for the manufacture of a medicament for the treatment, prevention or alleviation of an osteoclast related bone disease in a subject, which maybe a mammal, e.g. a human.

In one embodiment, the test cell comprises one or more chloride channels selected from the group consisting of ClC-3, ClC-6, ClC-7.

In a further embodiment, the osteoclast related bone disease is osteoporosis, osteolytic cancer invation, osteopetrosis, or Paget's disease of bone.

Preferred methods comprise screening the compounds so as to select compounds having the ability to block the chloride channel ClC-7 but not having the ability to block a chloride channel selected from the group consisting of chloride channels ClC-1, ClC-2, ClC-4, ClC-5, ClC-Ka and ClC-Kb.

In the context of this invention, the term "osteoclast elated bone disease" covers any deviation of bone resorption related to osteoclast associated diseases or disorders, such as osteoporosis, osteolytic cancer invation, osteopetrosis, or Paget's disease of bone.

In the context of this invention, the chloride channels of the ClC family are the chloride channels having the signature in the P1 region as described by Fahlke, C, in *Kidney Int* (2000) 57(3):780-6.

In the context of this invention, ClC-3 is the chloride channel of the ClC family as described by Borsani et al (Genomics (1995) 27(1) 131-141), and with GenBank Acc. No AF029348.

In the context of this invention, ClC-6 is the chloride channel of the ClC family as described by Brandt, S. et al (FEBS lett. (1995) 377 15-20), and with GenBank Acc. No AF209724.

In the context of this invention, ClC-7 is the chloride channel of the ClC family as described by Brandt, S. et al (FEBS lett. (19985) 377 15-20) and with GenBank Acc. No AF224741.

Functional analogues of the chloride channels CIC-3, CIC-6, and CIC-7 are chloride channels having substantially equivalent activity as compared to the unmodified chloride channel. Such analogues include splice variants, isoforms, homologues from other species.

In the test cells, the chloride channels may exist as dimer forms, either homodimers or heterodimers, such as a heterodimer of of ClC-3 and ClC-6.

In the context of this invention, a blocker of a chloride channel of the ClC family, such as CIC-3, ClG6, and ClG7, is a compound that blocks said chloride channel.

The ability of a compound to block a specific chloride channel, such as CIC-3, CIC-6, or CIC-7, can be measured as described in the method of example 4.

In one embodiment, the ability of a blocker of a chloride channel of the ClC family to block the ClC-3 shows an IC₅₀ value less than 100µM, preferably less than 10µM, and more preferably less than 1µM.

In a further embodiment, the ability of a blocker of a chloride channel of the ClC family to block the CIC-6 shows an IC₅₀ value less than 100µM, preferably less than 10µM, and more preferably less than 1µM.

In a still further embodiment, the ability of a blocker of a chloride channel of the ClC family to block the CIC-7 shows an IC₅₀ value less than 100µM, preferably less than 10µM, and more preferably less than 1µM.

In a further embodiment, the ability of a blocker of a chloride channel of the ClC family to block ClC-1, ClC-2, ClC-4, ClC-5, ClC-Ka, and ClC-Kb shows an IC₅₀ value higher than 1µM, preferably higher than 10µM, and more preferably higher than 100µM.

The chloride channel may or may not be endogenous to the test cell to be used in the method of screening, i.e. be a chloride channel naturally occurring in the cell.

Preferably, the chloride channel may be exogenous to the cell in question, and may in particular be introduced by recombinant DNA technology, such as transfection or infection. Such cells include human embryonic kidney (HEK) cells, in particular HEK 293 cells, Chinese hamster ovary 9CHO) cells, *Xenopus laevis* oocytes, or any other cell lines capable to chloride channels.

Examples of pharmaceutically acceptable addition salts of the compounds screened according to the invention include inorganic and organic acid addition salts such as the hydrochloride, hydrobromide, phosphate, nitrate, perchlorate, sulphate, citrate, lactate, tartrate, maleate, fumarate, mandelate, benzoate, ascorbate, cinnamate, benzenesulfonate, methanesulfonate, stearate, succinate, glutamate, glycollate, toluene-p-sulphonate, formate, malonate, naphthalene-2-sulphonate, salicylate and the acetate. Such salts are formed by procedures well known in the art.

Other acids such as oxalic acid, while not in themselves pharmaceutically acceptable, may be useful in the preparation of salts useful as intermediates in obtaining compounds of the invention and their pharmaceutically acceptable acid additions salts.

The screened compounds may exist in unsolvated as well as insolvated forms with pharmaceutically acceptable solvents such as water, ethanol and the like. In general, the solvated forms are considered equivalent to the unsolvated forms for the purposes of this invention.

### Pharmaceutically Acceptable Salts

The chemical compounds may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconite, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrte, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

### Prodrugs

The substance screened according to the invention may be administered as such or in the form of a suitable prodrug thereof. The terem "prodrug" denoates a bioreversible derivate of the drug, the bioreversible derivate4 being therapeutically substantially inactive per se but being able to convert in the body to the active substance by an enzymatic or non-enzymatic process.

Thus, examples of suitable prodrugs of the substances include compounds obtained by suitable bioreversible derivatization of one or more reactive or derivatizable groups of the parent substance to result in a bioreversible derivative. The derivatization may be performed to obtain a higher bioavailability of the active substance, to stabilize an otherwise unstable active subtstance, to increase the lipophilicity of the substance administered, etc.

Examples of types of substances which may advantageously be administered in the form of prodrugs are carboxylic acids, other acidic groups and amines, which may be rendered more lipophilic by suitable bioreversible derivatization. As examples of suitable groups may be mentioned bioreversible esters or bioreversible amides. Amino acids are typical examples of substances which, in their unmodified form, may have a low absorption upon administration. Suitable prodrug derivatives of amino acids will be one or both of the above-mentioned types of bioreversible derivatives.

### Method of Screening

Screening for the ability of a compound of block one or more chloride channels from the ClC family can be performed by a multitude of techniques well known in the art.

Examples of available screening methods are conventional electrophysiological methods such as patch-clamp techniques, or convention spectroscopic methods such as FLIPR assay (Fluorescence Image Plate Reader; available from Molecular Devices), or VIPR (voltage ion probe reader, available from Aurora).

These methods generally comprise monitoring the membrane potential of the chloride channel containing cell in order to identify changes in the membrane potential caused by the action of the compound.

Other screening methods include ligand binding assays, Cl-flux based assays and oocyte voltage clamp after expression of ClC cDNA in an appropriate heterologous system.

### Pharmaceutical Compositions

While a chemical compound for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

Pharmaceutical compositions may be formulated comprising the chemical compound, or a pharmaceutically acceptable salt or derivative thereof, together with one or more pharmaceutically acceptable carriers therefor, and, optionally, other therapeutic and/or prophylactic ingredients, known and used in the art. The carrier(s) must be "acceptable" in the sense of being compatible with the other ingredients of the formulation and not harmful to the recipient thereof.

The pharmaceutical composition may be administered by any convenient route which suit the desired therapy. Preferred routes of administration include oral administration, in particular in tablet, in capsule, in dragé, in powder, or in liquid form, and parenteral administration, in particular cutaneous, subcutaneous, intramuscular, or intrafvenous injection. The pharmaceutical composition may be prepared by the skilled person using standard and conventional techniques appropriate to the desired formulation. When desired, compositions adapted to give sustained relase of the active ingredient may be employed.

Further details on techniques for formulation and administration may be found in the latest edition of Remington's Pharmaceutical Sciences (Maack Publishing Co., Easton, PA).

The actual dosage depend on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.01 to about 500 mg of active ingredient per individual dose, preferably of from about 0.1 to about 100 mg, most preferred of from about 1 to about 10 mg, are suitable for therapeutic treatments.

The active ingredient may be administered in one or several doses peer day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

The following examples will illustrate the invention further, however they are not to be construed as limiting.

### EXAMPLES

### Example 1

### Identification of ClC channels of osteoclasts

CIC-3 (GenBank Acc. No AF029348), CIC-6 (GenBank Acc. No AF209724), and CIC-7 (GenBank Acc. No AF224741), channels were identified in human osteoclasts (developed from macrophages, OsteoPro) using a RT-PCR based strategy as described below.

### Methods

Purification of total RNA from osteoclasts (using RNeasy kit, Qiagen; or standard methods such as described in Ausubel, FM, Brent, R, Kingston, RE, Moore, DD, Seidman, JG, Smith, JA and Struhl, K eds. (2000) Current protocols in Molecular Biology Vol 1, Wiley & Sons)

Sequence specific RT-PCR using PCR primers specific for the different subtypes of Cl⁻ channels of the ClC, CICA and CLIC families.
RT mix
3 µg total RNA or 30-150 ng mRNA
2,5 µM oligo dT final concentration
Denaturate 10 min at 70°C, place then at ice.

| Add: | |
|---|---|
| 1 mM | dNTP final concentration (Pharmacia) |
| 5 mM | MgCl₂ final concentration (Gibco BRL) |
| 1X | PCR buffer without MgCl₂(Gibco BRL) |
| 20 units | Rnase inhibitor (Perkin Elmer) |

H₂O to 20 µl final volumen
Anneal 10 min at room temperature
50 units RT Mulv (Perkin Elmer)
Incubate 1 hour at 42°C and 15 min 70°C
RT-PCR
1/10 of the RT mix is used in the RT-PCR reaction

| | |
|---|---|
| 2 µl | RT mix |
| 250 µM | dNTP final concentration |
| 0.5 µM | sense and antisense primer final concentration |
| 1.5 mM | MgCl₂ final concentration (Gibco BRL) |
| 1X | PCR buffer without MgCl₂ (Gibco BRL) |
| 2.5 U | Taq polymerase (Gibco BRL) |

H₂O to 20 µl final volumen
Using the following sequence specific primers:

| **Specificity** | **Sense Antisense** | **Position** |
|---|---|---|
| CIC-3 | gcagaattaatcataggccaagca atactttggaaagaggtaggaaaa | 580 |
| | | 909 |
| CIC-6 | ctcctgggctttaacttgaccttc aaactggatcttccgtaaggagat | 397 |
| | | 681 |
| CIC-7 | ggtgtctgccggggtcttcatcc gagccgggcaggctgggtgtc | 1524 |
| | | 2003 |

PCR profile
94°C 2 min. 94°C 1 min, 60/66°C 1 min, 72°C 1 min repeated 29 times. 72°C 5 min.
4°C for ever.

### Northern blot using standard methods

Results obtained by RT-PCR may be validated by northern blot. 10 µg human osteoclast total RNA is blotted on hybond membranes (Amersham Pharmacia Biotech) and prehybridized for 30 min at 65°C in ExpressHyb (Clontech). CIC-3, CIC-6 and ClC-7 specific P³²labeled DNA probes are generated using random priming (Amersham Pharmacia Biotech) and hybridized to the blotted membranes for 16 hours at 65°C in ExpressHyb. The hybridized membrane is washed and analyzed by auto adiography.

### Example 2

### Expression of CIC-3, CIC-6, and ClC-7 in HEK293 cells

The CIC-3, CIC-6, and CIC-7 were recombinant expressed in HEK293 cells using standard methods based on lipofectamine transfection (Life Technologies).

Briefly, CIC-3, CIC-6, and CIC-7 was excised from pCRII-TOPO (Invitrogen), and subcloned into a mammalian/oocyte expression vector generating expression vectors, named pZOOM_ClC3, pZOOM_ClC6, and pZOOM_ClC7.

HEK293 tissue culture cells were grown in DMEM (Dulbecco's Modified Eagle Medium) supplemented with 10% FCS (foetal calf serum) at 37°C in 5% CO₂. One day prior to transfection, 10⁶ cells were plated in a cell culture T25 flask. The following day, cells were transfected using lipofection (20 µL Lipofectamin™, Life Technologies, with 2.5 µg of the pZOOM_ClC3, pZOOM_ClC6, and pZOOM_ClC7 expression vectors respectively in a total volume of 540 µL).

The lipofection mixture was overlaid on the cells and incubated at 37°C for 5 hours. The cells were then rinsed with regular media and grown for 72 hours in DMEM, 10% FCS at 37°C in 5% CO₂.

72 hours post transfection, cells transfected with pZOOM were selected in media supplemented with 0.5 µg/ml G418. Single clones were picked and propagated in selection media until sufficient cells for freezing were available. Hereafter the cells were cultured in regular medium without selection agent.

Expression of functional CIC-3, CIC-6, and CIC-7 channels was verified by patch-clamp measurements.

### Example 3

### Patch clamp screening for compounds

The following method can be used for screening compounds for chloride channel blocking activity.

Experiments are carried out on one of several patch-clamp set-ups. Cells plated on coverslips are placed in a 15 µl perfusion chamber (flowrate ~1 ml/min) mounted on an IMT-2 microscope equipped with Nomarski or Hoffmann optics. The microscopes are placed on vibration-free tables in grounded Faraday cages. All experiments are performed at room temperature (20 - 22°C). EPC-9 patch-clamp amplifiers (HEKA-electronics, Lambrect, Germany) are connected to Macintosh computers via ITC16 interfaces. Data are stored directly on the harddisk and analysed by the IGOR software (WaveMetrics, Lake Oswega, USA).

The whole-cell configuration of the patch clamp technique is applied. The tip of a borosilicate pipette (resistance 2-4 MΩ) is gently (remote control system) placed on the cell membrane. Light suction results in a giga seal (pipette resistance increases to more than 1 GΩ) and the cell membrane is then ruptured by more powerful suction. Cell capacitance is electronically compensated and the resistance between the pipette and the cell interior (the series resistance, Rs) is measured and compensated for. Usually the cell capacitance ranges from 5 to 20 pF (depending on cell size) and the series resistance is in the range 3 to 6 MΩ. Rs- as well as capacitance compensation are updated during the experiments (before each stimulus).

All experiments with drifting Rs-values are discharged. Leak-subtractions are not performed.

The extracellular (bath) solution contains: 144 mM KCI, 2 mM CaCl₂, 1 mM MgCl₂, 10 mM HEPES (pH = 7.4). Test compounds are dissolved in DMSO from stock solution and then diluted to a final concentration of about 10 µM in the extracellular solution. The concentration of CaCl₂ is 7.6 mM and that of MgCl₂ is 1.2 mM to give calculated free concentrations of 300 nM and 1 mM, respectively.

### Quantification

After establishment of the whole-cell configuration, voltage-ramps (usually -100 to +100 mV) are applied to the cell every 5 sec. A stable baseline current is obtained within a period of 100-300 seconds, and the compounds are then added by changing to an extracellular solution containing the compound to be tested. Very little endogen current is activated under these circumstances in native HEK293 cells.

### Example 4

### Northern blot for clc-type channels

Samples of 5 µg total RNA from human fetal brain (Stratagene), 10 µg total RNA from human osteoclast cultures differentiated in the presence of RANKL for one week and 10 µg total RNA from human osteoclast cultures differentiated in the presence of RANKL for two weeks were separated on a denaturing formaldehyde gel, photographed and transferred to Hybond N membranes (Amersham) using capillary blotting.

Three membranes containing identical amounts of RNA were produced and probed with 32P-labeled PCR-fragments (Rediprime II random labelling kit, Amersham) for human CIC-7 (bases 1524-2026), human CIC-6 (bases 1171-1624) or human ClC-3 (bases 242-651), respectively. Prehybridisation and hybridisation were performed with Ambion's Ultrahyb hybridisation buffer at 42°C for 30 min and overnight, respectively, followed by two short washes with 2x SSC/0.1 % SDS and two 15 min washes with O.1x SSC/0.1% SDS (20x SSC: 3M NaCl, 0.3 M sodium citrate, pH 7).

Membranes were wrapped in plastic foil and exposed on Kodak Biomax ML film for up to three weeks.

An approximately 4 kb transcript of human CIC-7 could be detected in brain and both human osteoclast preparations. The strongest signal was observed in the most differentiated osteoclasts (two weeks differentiation in the presence of RANKL). A weak 6.2 kb message of human CIC-6 could be observed in human fetal brain, but not in human osteoclasts, even after prolonged exposure. Human CIC-3 was not detectable in either sample.

The size of ClC-7 and ClC-6 transcripts correspond very well to those described in: Brandt and Jentsch (1995) FEBS Letters 377, 15-20 (4.2 kb and 6 kb, respectively).

Thus, ClC-7 is the only ClC-type channel among the three tested detectable in human osteoclasts. Furthermore, the m RNA seems to be upregulated during osteoclast differentiation, indicating the relevance of this channel in osteoclast physiology. CIC-6 and CIC-3, however, do not seem to be significantly expressed in osteoclasts, but can be detected by the more sensitive RT-PCR method.

## Claims

1. A method for screening chemical compounds for activity in the treatment, prevention, or alleviation of an osteoclast related bone disease in a subject, which method comprises screening compounds in a test cell so as to select compounds having the ability to block one or more channels of the ClC family.

2. A method as claimed in claim 1, comprising screening the compounds so as to select compounds having the ability to block one or more of the chloride channels ClC-3, ClC-6 and ClC-7.

3. A method as claimed in claim 1, comprising screening the compounds so as to select compounds having the ability to block the chloride channel ClC-7.

4. A method as claimed in claim 3, comprising screening the compounds so as to select compounds having the ability to block the chloride channel ClC-7 but not having the ability to block a chloride channel selected from the group consisting of chloride channels ClC-1, ClC-2, ClC-4, ClC-5, ClC-Ka and ClC-Kb.

5. A method as claimed in claim 3, comprising screening the compounds so as to select compounds having the ability to block specifically the chloride channel ClC-7.

6. A method as claimed in claim 1 or claim 2, which method comprises the following steps:
• providing a test cell comprising one or more chloride channels of the ClC family;
• subjecting the test cell to the action of the chemical compound; and
• measuring the ability of the compound to block the one or more chloride channels.

7. A method as claimed in Claim 6, wherein the test cell comprises one or more chloride channels selected from the group consisting of ClC-3, C1C-6 and ClC-7.

8. A method as claimed in claim 6, which screens a chemical compound for activity in blocking specifically the chloride channel ClC-7 in which the test cell comprises the chloride channel ClC-7 or a functional analogue of the chloride channel ClC-7 which is such that compounds which specifically block said functional analogue also specifically block the chloride channel ClC-7.

9. A method as claimed in claim 8, wherein said test cell comprises the chloride channel ClC-7.

10. A method as claimed in any one of Claims 1 to 9, wherein the osteoclast related bone disease is osteoporosis, osteolytic cancer invasion, osteopetrosis, or Paget's disease of bone.

## Patentansprüche

1. Verfahren zum Screening von chemischen Verbindungen für Aktivität bei der Behandlung, Verhinderung oder Linderung einer durch Osteoklasten bedingten Knochenkrankheit in einem Patienten, welches Verfahren das Screening von Verbindungen in einer Testzelle umfasst, um Verbindungen zu selektieren, die die Fähigkeit aufweisen, einen oder mehrere Kanäle der ClC-Familie zu blockieren.

2. Verfahren nach Anspruch 1, umfassend das Screening der Verbindungen, um Verbindungen zu selektieren, die die Fähigkeit aufweisen, einen oder mehrere von den Chloridkanälen ClC-3, ClC-6 und ClC-7 zu blockieren.

3. Verfahren nach Anspruch 1, umfassend das Screening der Verbindungen, um Verbindungen auszuwählen, die die Fähigkeit aufweisen, den Chloridkanal ClC-7 zu blockieren.

4. Verfahren nach Anspruch 3, umfassend das Screening der Verbindungen, um Verbindungen auszuwählen, die die Fähigkeit aufweisen, den Chloridkanal ClC-7 zu blockieren, die aber nicht die Fähigkeit haben, einen Chloridkanal zu blockieren, ausgewählt aus der Gruppe, bestehend aus den Chloridkanälen ClC-1, ClC-2, CIC-4, ClC-5, ClC-Ka und ClC-Kb.

5. Verfahren nach Anspruch 3, umfassend das Screening der Verbindungen, µm Verbindungen auszuwählen, die die Fähigkeit aufweisen, spezifisch den Chloridkanal ClC-7 zu blockieren.

6. Verfahren nach Anspruch 1 oder Anspruch 2, welches Verfahren die folgenden Schritte umfasst:
• Bereitstellen einer Testzelle, die einen oder mehrere Chloridkanäle der CIC-Familie umfasst;
• Aussetzen der Testzelle an die Wirkung der chemischen Verbindung; und
• Messen der Fähigkeit der Verbindung, um den einen oder die mehreren Chloridkanäle zu blockieren.

7. Verfahren nach Anspruch 6, worin die Testzelle einen oder mehrere Chloridkanäle umfasst, ausgewählt aus der Gruppe, bestehend aus CIC-3, CIC-6 und CIC-7.

8. Verfahren nach Anspruch 6, das eine chemische Verbindung für Aktivität beim spezifischen Blockieren des Chloridkanals CIC-7 screent, in dem die Testzelle den Chloridkanal CIC-7 oder ein funktionelles Analogon des Chloridkanals ClC-7 umfasst, welches so ist, dass Verbindungen, die dieses funktionelle Analogon spezifisch blockieren, ebenfalls spezifisch den Chloridkanal ClC-7 blockieren.

9. Verfahren nach Anspruch 8, worin diese Testzelle den Chloridkanal ClC-7 umfasst.

10. Verfahren nach irgendeinem der Ansprüche 1 bis 9, worin die durch Osteoklasten bedingte Knochenkrankheit Osteoporose, Invasion von osteolytischem Krebs, Osteopetrose oder Paget's Krankheit des Knochens ist.

## Revendications

1. Procédé de criblage de composés chimiques en vue d'une activité dans le traitement, la prévention ou le soulagement d'une maladie osseuse associée aux ostéoclastes chez un sujet, lequel procédé comprend le criblage de composés dans une cellule d'essai afin de sélectionner des composés ayant la capacité de bloquer un ou plusieurs canaux de la famille ClC.

2. Procédé selon la revendication 1, comprenant le criblage des composés afin de sélectionner des composés ayant la capacité de bloquer un ou plusieurs des canaux à chlorure ClC-3, ClC-6 et ClC-7.

3. Procédé selon la revendication 1, comprenant le criblage des composés afin de sélectionner des composés ayant la capacité de bloquer le canal à chlorure ClC-7.

4. Procédé selon la revendication 3, comprenant le criblage des composés afin de sélectionner des composés ayant la capacité de bloquer le canal à chlorure ClC-7, mais n'ayant pas la capacité de bloquer un canal à chlorure choisi dans le groupe constitué par les canaux à chlorure ClC-1, ClC-2, ClC-4, ClC-5, ClC-Ka et ClC-Kb.

5. Procédé selon la revendication 3, comprenant le criblage des composés afin de sélectionner des composés ayant la capacité de bloquer spécifiquement le canal à chlorure ClC-7.

6. Procédé selon la revendication 1 ou la revendication 2, ledit procédé comprenant les étapes suivantes consistant à :
fournir une cellule d'essai comprenant un ou plusieurs canaux à chlorure de la famille ClC ;
soumettre la cellule d'essai à l'action du composé chimique ; et
mesurer la capacité du composé à bloquer le ou les canaux à chlorure.

7. Procédé selon la revendication 6, dans lequel la cellule d'essai comprend un ou plusieurs canaux à chlorure choisis dans le groupe constitué par le ClC-3, le ClC-6 et le ClC-7.

8. Procédé selon la revendication 6, qui crible un composé chimique en vue d'une activité bloquant spécifiquement le canal à chlorure ClC-7 dans lequel la cellule d'essai comprend le canal à chlorure ClC-7 ou un analogue fonctionnel du canal à chlorure ClC-7 qui est tel que les composés qui bloquent spécifiquement ledit analogue fonctionnel bloquent également spécifiquement le canal à chlorure ClC-7.

9. Procédé selon la revendication 8, dans lequel ladite cellule d'essai comprend le canal à chlorure ClC-7.

10. Procédé selon l'une quelconque des revendications 1 à 9, dans lequel la maladie osseuse associée aux ostéoclastes est l'ostéoporose, l'invasion d'un cancer ostéolytique, l'ostéopétrose ou la maladie osseuse de Paget.
